(19) Europäisches Patentamt
European Patent Office
Office européen des brevets

(11) Veröffentlichungsnummer: **0 185 210 B1**

# (12) EUROPÄISCHE PATENTSCHRIFT

(45) Veröffentlichungstag der Patentschrift: **12.06.91**

(51) Int. Cl.$^5$: **A61K 37/02**

(21) Anmeldenummer: **85114680.3**

(22) Anmeldetag: **19.11.85**

## (54) Verwendung von Dipeptidderivaten für die Herstellung von Arzneimitteln zur Behandlung von an amyotropher Lateralsklerose Erkrankten.

(30) Priorität: **18.12.84 DE 3446127**
**22.12.84 DE 3447260**

(43) Veröffentlichungstag der Anmeldung:
**25.06.86 Patentblatt 86/26**

(45) Bekanntmachung des Hinweises auf die Patenterteilung:
**12.06.91 Patentblatt 91/24**

(84) Benannte Vertragsstaaten:
**AT BE CH DE FR GB IT LI LU NL SE**

(56) Entgegenhaltungen:
**FR-A- 2 347 338**
**US-A- 4 386 073**

**THE LANCET, 9. Juli 1983, Seiten 73-75; W. KING ENGEL et al.: "Effect on weakness and spasticity in amyotrophic lateral sclerosis of thyrotropin-releasing hormone"**

**PROCEEDINGS OF THE B.P.S., Band 70, Nr. 1, 9.-11. April 1980, Seiten 81P-82P; A.R. GREEN et al.: "Behavioural effects of central and peripheral injection of two synthetic analogues of TRH"**

(73) Patentinhaber: **Grünenthal GmbH**
**Zieglerstrasse 6**
**W-5100 Aachen(DE)**

(72) Erfinder: **Flohé, Leopold, Prof. Dr.**
**Lenzbach 24**
**W-5106 Roetgen(DE)**
Erfinder: **Barth, Hans, Dr. Dr.**
**Taubengasse 12**
**W-5100 Aachen(DE)**

**EP 0 185 210 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

PROCEEDINGS OF THE B.P.S., Band 74, Nr. 4, 16.-18. September 1981, Seiten 868P-869P; E.C. GRIFFITHS et al.: "Locomotor stimulant actions of some TRH analogues"

NEUROPHARMAC., Band 20, Nr. 10, 1981, Seiten 947-957, Pergamon Press Ltd; D.J. HEAL et al.: "Behavioural effects of central and peripheral injection of various analogues and metabolites of thyrotropin releasing hormone (TRH)"

THE LANCET, 23. Februar 1980, Seiten 418-419; I. SOBUE et al.: "Effect of thyrotropin-releasing hormone on ataxia of spinocerebellar degeneration"

PEPTIDES, Band 2, Nr. 2, 1981, Seiten 131-135, ANKHO International Inc.; S. YEHUDA et al.: "Effects of TRH and PS-24 on colonic temperature and motor activity of rats: Possible role of dopamine"

## Beschreibung

Die amyotrophe Lateralsklerose stellt eine Erkrankung des Nervensystems dar, bei der die motorischen Nerven, die die Befehle für alle Körperbewegungen vom Gehirn an die Muskeln leiten, zerstört werden. Aufgrund des Wegfalls dieser Bewegungsbefehle tritt ein Muskelschwund ein, wodurch mit dem Fortschreiten der Krankheit die verschiedenen Körperfunktionen ausfallen und Greifen, Gehen, Sprechen und schließlich sogar Atmen unmöglich wird, obwohl das Gehirn als solches voll funktionsfähig bleibt. Der Tod des Patienten tritt durchschnittlich innerhalb von 5 Jahren nach Auftreten der ersten Symptome ein. Die Ursachen dieser Krankheit sind unbekannt, und bis heute ist weder eine Heilung noch eine länger andauernde Linderung der Beschwerden der amyotrophen Lateralsklerose (nachstehend abgekürzt zu "ALS") möglich gewesen.

Es wurde nun gefunden, daß durch Verabreichung von Arzneimitteln, welche Dipeptidderivate der folgenden allgemeinen Formel I

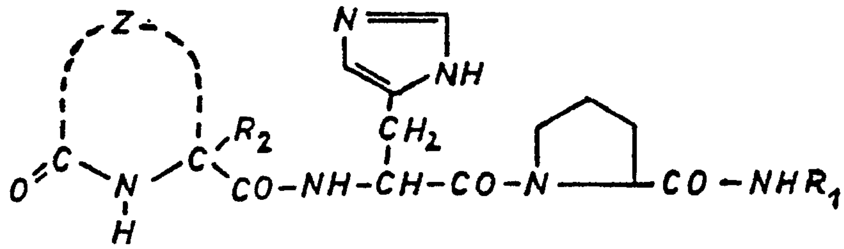

(I)

in der $R_1$ ein Wasserstoffatom, einen Alkylrest mit bis zu 6 Kohlenstoffatomen, einen Cyclohexylrest oder einen Benzylrest bedeutet, und in der Z für eine der (mit der durch den Stern markierten Bindung an die CO-Gruppe im Ring angreifenden) Gruppen

(a) 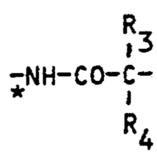 oder (b) 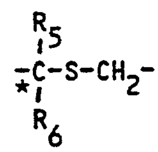

steht, $R_2$ zusammen mit $R_3$ für eine zusätzliche Bindung oder, wenn Z die Gruppe(b) darstellt, für ein Wasserstoffatom steht und in der $R_4$ und $R_5$ gleich oder verschieden sind und für Wasserstoffatome oder für Alkylreste mit 1 bis 3 Kohlenstoffatomen stehen und $R_5$ auch Phenyl bedeuten kann und $R_6$ ein Wasserstoff atom oder Methyl bedeutet,
oder deren pharmazeutisch anwendbare. Salze mit Säuren
enthalten, bei ALS-Patienten eine Besserung der klinischen Symptomatik erreicht werden kann, die auch noch längere Zeit nach Absetzen der Medikation anhält.

Bevorzugte Verbindungen der allgemeinen Formel I entsprechen den allgemeinen formeln

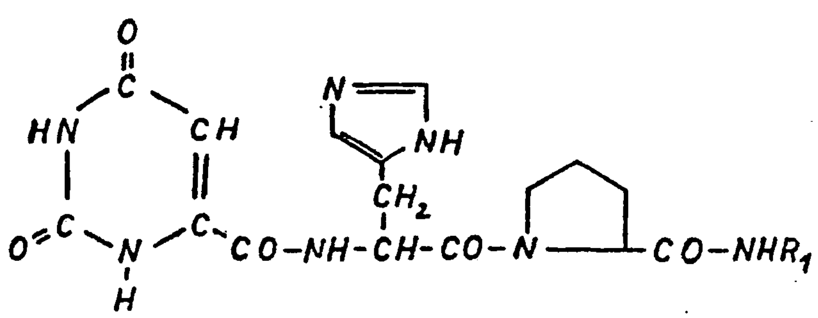

(Ia)

oder

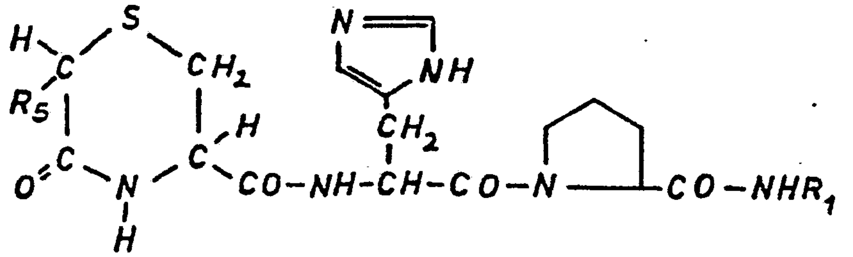 (Ib)

in denen $R_1$ und $R_5$ die oben angegebenen Bedeutungen haben.

Vorzugsweise steht in den allgemeinen Formeln I, Ia bzw. Ib $R_1$ für ein Wasserstoffatom.

Besonders bevorzugte Verbindungen der allgemeinen Formel I sind Orotyl-L-histidyl-L-prolinamid und 5-Oxo-6-methyl-thiomorpholin-3-(L)-carbonyl-L-histidyl-L-prolinamid.

Die Verbindungen der allgemeinen Formel I und ihre Herstellung sind z. B. von Schwertner et al. in "Structure and Activity of Natural Peptides" (Editors W. Voelter und G. Weitzel) Walter de Gruyter-Verlag, Berlin - New York 1981, Seiten 397 - 415 sowie z. B. in den deutschen Patentschriften 2 449 167 und 2 615 455 beschrieben worden.

Aus diesen Literaturstellen ist bekannt, daß die Verbindungen der allgemeinen Formel I nach parenteraler oder auch oraler Verabreichung lang anhaltende zentral erregende Wirkungen ausüben und nur eine geringe Toxizität besitzen. Während sie in einer Anzahl von pharmakologischen Testen qualitativ vergleichbare Eigenschaften wie das ebenfalls bekannte TRH (L-Pyroglutamyl-L-histidyl-L-prolinamid) zeigten (vgl. u. a. Flohé et al. in "Thyrotropin-Releasing Hormone", Eds. E.C. Griffiths und G.W. Bennett, Raven Press, New York 1983, Seiten 327 bis 340), unterscheiden sie sich in anderen pharmakologischen Eigenschaften von TRH (vgl. Yehuda et al., Peptides 2, 1981, Seiten 131 bis 135).

Weiterhin ist zu beachten, daß (vgl. Flohé et al., loc. cit.) die Verbindungen dem allgemeinen Formel I gegen eine enzymatische Metabolisierung wesentlich stabiler als TRH sind.

Für (synthetisch hergestellte) Verbindungen der allgemeinen Formel I, in denen die Gruppe $NHR_1$ durch OH ersetzt wurde, sind Wirkungsdauer und -stärke im Vergleich zu den Amiden der allgemeinen Formel I aber auch im Vergleich zu TRH stark herabgesetzt, wie Griffiths et al. (Proc. of the B.P.S. 74 Nr. 4, 1981, Seiten 868P bis 869P) fanden, d. h. Deaminoverbindungen dieser Struktur müssen als praktisch unwirksam angesehen werden.

Nach den Angaben der genannten und weiterer Literaturstellen können aufgrund der ermittelten pharmakologischen Eigenschaften die Verbindungen der allgemeinen Formel I als Psychostimulantien bzw. als Antidepressiva eingesetzt werden, wobei zusätzlich von erheblichem Vorteil ist, daß für die Verbindungen der allgemeinen Formel I (anders als für TRH) die für die genannten Indikationen als unerwünschte Nebenwirkungen anzusehenden endocrinologischen Wirkungen nur schwach ausgeprägt sind (vgl. Flohé et al., loc. cit. und FR-A-2 347 338, Seite 3, Zeilen 30 bis 35).

Eine Therapie der ALS ist bisher nicht bekannt geworden, jedoch berichteten Engel et al. in The Lancet, July 9, 1983, Seiten 73 bis 75 über kurzfristige Effekte auf Schwäche und Spastik von ALS-Patienten bei Verabreichung hoher intravenösen von TRH. Diese unter zum Teil sehr starken Nebenwirkungen auftretenden Effekte konnten während der Infusion von TRH und für etwa eine Stunde danach, in einigen Fällen in schwach ausgeprägtem Maße für bis zu 20 Stunden beobachtet werden. Offen blieb, ob diese Effekte durch TRH oder durch seine Metaboliten (wie Histidinprolindiketopiperazin, Deamino-TRH, Pyroglutaminsäure, Histidin, Prolin oder deren Abbauprodukte Glutaminsäure, $\gamma$-Aminobuttersäure oder Glycin) bewirkt wurden.

Die von Engel et al. geschilderten Befunde haben bisher nicht zur Einführung bzw. generelleren Anwendung von TRH oder eines der als potentiell für die Effekte verantwortlich aufgeführten Metaboliten zur ALS-Therapie geführt, so daß nach wie vor keine Therapiemöglichkeit für die ALS gegeben ist.

Die bekannten Indikationsbereiche der Verbindungen der allgemeinen Formel I betreffen solche Krankheiten, bei denen das Gehirn als solches, nicht aber beispielsweise die motorischen Nerven erkrankt sind. Wie einleitend erläutert, sind demgegenüber bei der ALS bei Patienten mit voll funktionsfähigem, gesundem Gehirn die motorischen Nerven erkrankt. Es war daher nicht vorauszusehen, ob und daß die Dipeptidderivate der allgemeinen Formel I bzw. diese enthaltende Arzneimittel bei ALS, also einem völlig anderen Krankheitsgeschehen unbekannter Genese einen günstigen Einfluß auf die krankheitsbedingten Beschwerden haben könnten. Vielmehr mußte man es auch unter Berücksichtigung der von Engel et al. nach Verabreichung hoher TRH-Dosen beobachteten unbefriedigenden Befunde (die außerdem nicht

bestimmten Verbindungsklassen zugeordnet werden konnten) als abwegig ansehen, die Verbindungen der allgemeinen Formel I mit völlig anderem Metabolisierungsverhalten und anderen biologischen Eigenschaften als TRH hinsichtlich ihrer Wirkung auf die ALS-Symptome zu untersuchen.

Aufgrund der Ergebnisse derartiger für den Fachmann abwegig erscheinender Versuche bezieht sich daher die Erfindung auf die Verwendung von Dipeptidderivaten der allgemeinen Formel I für die Herstellung von Arzneimitteln zur Behandlung von an amyotropher Lateralsklerose Erkrankten.

Da diese Verbindungen der allgemeinen Formel I chemisch recht stabil sind, wirft ihre Einarbeitung in pharmazeutische Zubereitungsformen für den Fachmann keinerlei Probleme auf. Zur Erläuterung sei dennoch aber folgendes gesagt:
Die Applikationsart richtet sich primär nach dem bei dem zu behandelnden Einzelpatienten beobachteten Krankheitsbild. Vorzugsweise kommen solche Arzneimittel bzw. Wirkstoffzubereitungen in Betracht, die eine Verabreichung der Verbindungen der allgemeinen Formel I an die ALS-Patienten in Form von Infusionen, insbesondere auch intrathekalen Infusionen, bzw. intravenösen oder auch intramuskulären oder intraperitonealen Injektionen gestatten. Solche an sich bekannten Zubereitungsformen bestehen insbesondere aus der in trockener, steriler Form (gegebenenfalls als Lyophilisat) in einem Fläschchen mit durchstechbarem Stopfen abgefüllten Wirkstoffmenge. Durch Zugabe eines geeigneten Lösungsmittels, wie z. B. einer sterilen, isotonischen wässrigen Lösung von Kochsalz, Glucose, Inosit oder dergleichen, worin die Verbindungen der allgemeinen Formel I leicht löslich sind, erhält man zur Injektion oder Infusion einsetzbare Arzneimittellösungen.

Gut geeignete Applikationsformen sind auch Sprays zur intranasalen oder oralen Applikation bzw. zur Verabreichung der Substanzen über die Bronchien, die in üblicher Form hergestellt werden können.

Insbesondere zur Aufrechterhaltung der beispielsweise durch Verabreichung von Infusionsformen einleitend erreichten Effekte kommen auch oral anwendbare Zubereitungsformen der Verbindungen der allgemeinen Formel I, wie Tabletten, Dragees, Kapseln, Granulate, Tropfen und Säfte bzw. Sirupe in Betracht. Auch diese Zubereitungsformen sind an sich bekannt bzw. können in üblicher Weise hergestellt werden.

So kann man beispielsweise zur Herstellung von Tabletten oder Dragees, die jeweils 20 mg einer Verbindung der allgemeinen Formel I enthalten, so vorgehen, daß 20 g der Wirksubstanz mit 35 g Maisstärke, 10 g kolloidaler Kieselsäure, 5 g Magnesiumstearat und gegebenenfalls Zusätzen von Farbstoffen und/oder Aromen vermischt, granuliert, getrocknet und zu 1000 Tabletten gepreßt werden, die dann gewünschtenfalls noch mit einem Überzug versehen bzw. dragiert werden.

Zur Herstellung von jeweils 20 mg der Verbindung der allgemeinen Formel I enthaltenden Kapseln kann man in üblicher Weise z. B. 20 g Wirkstoff mit 376 g Lactose vermischen, das Gemisch nach Befeuchten mit einer wässrigen Lösung von 4 g Gelatine granulieren, trocknen und in 1000 Hartgelatine-Kapseln füllen.

Nasentropfen, die gegegebenenfalls auch in Sprayform appliziert werden können, kann man z. B. in an sich bekannter Weise dadurch erhalten, daß man die Verbindung der allgemeinen Formel I in einer isotonen Wässrigen Lösung von Natriumchlorid, Mannit, Sorbit oder Inosit löst und z. B. Polyvinylpyrrolidon oder Polyvinylalkohol als Haftmittel und/oder ein Konservierungsmittel,wie z. B. p-Hydroxybenzoesäuremethylester oder Benzylalkohol, zufügt.

Einen Wirkstoff der allgemeinen Formel I enthaltende Suppositorien kann man z. B. durch Schmelzen von 95 g einer handelsüblichen Suppositorienmasse bei 40 - 45° C, Zugabe von 3 g Salicylsäure oder Mandelsäure, anschließendes Einrühren von 2 g des Wirkstoffes und Einfüllen des Gemisches in Suppositorienformen herstellen.

In vielen Fällen kommen auch percutane Applikationszubereitungen (die z. B. die Wirkstoffe in einem Depot in gelöster Form, gegebenenfalls unter Zusatz von bekannten, die Hautpenetration fördernden Mitteln wie N-Alkyllactamen enthaltende Pflaster in Betracht.

Die vorstehend beschriebenen oral, rektal, percutan oder intramuskulär anwendbaren Zubereitungsformen können vorzugsweise in ebenfalls an sich bekannter Weise auch so hergestellt Werden, daß daraus nach der Applikation die Verbindung der allgemeinen Formel I verzögert freigesetzt wird, um so über einen längeren Zeitraum (beispielsweise 24 Stunden) eine gleichförmige Versorgung des Patienten mit dem Wirkstoff zu gewährleisten.

Die zur Linderung der Beschwerden von ALS-Patienten erforderlichen Dosen an Verbindungen der allgemeinen Formel I richten sich - abgesehen von der Applikationsart bzw. -form - insbesondere nach dem bei Therapiebeginn bestehenden Krankheitsstadium und sind vom Arzt individuellzu ermitteln. Als Anhaltspunkt kann davon ausgegangen werden, daß der in Betracht zu ziehende Dosierungsbereich für die Applikation mit Hilfe der Infusionstechnik bei etwa 5 bis 100 mg pro Tag liegt.

Die überraschende Wirkung der Verbindungen der allgemeinen Formel I bzw. der diese enthaltenden Arzneimittel bei an ALS Erkrankten ergibt sich aus der folgenden Schilderung von Ergebnissen aus klinischen Therapiestudien.

Ein Patient mit ALS-bedingter Störung der Muskeln des Sprech- und Kauapparates erhielt an vier aufeinanderfolgenden Tagen täglich drei Kurzinfusionen (innerhalb von je 30 Minuten in Abständen von je 4 Stunden) mit steigenden Mengen von Orotyl-L-histidyl-L-prolinamid. Der Wirkstoff war in lyophilisierter Form in Durchstechfläschchen, die 12 mg Mannit enthielten, mit je 6 mg Substanz abgefüllt und wurde zur Infusion in 100 ml steriler, isotonischer Kochsalzlösung gelöst. Dabei betrugen die applizierten Gesamtdosen am 1. Tag 18 mg, am 2. Tag 36 mg, am 3. Tag 54 mg und am 4. Tag 72 mg. Es zeigten sich ausgeprägte Wirkungen auf die bulbäre Symptomatik, und der Patient war beispielsweise wieder in der Lage zu sprechen. Die Nebenwirkungen dieser Behandlung waren unbedeutend und angesichts der günstigen Wirkung, die nach Absetzen der Medikation noch einige Tage anhielt, zu vernachlässigen.

Ein ALS-Patient mit achtmonatigem Krankheitsverlauf konnte nach gleicher Therapie Wie oben angegeben erstmals wieder die Arme über die Horizontale erheben, ohne Stock auf freier Strecke gehen, besser Treppen steigen, und er wurde in den alltäglichen Verrichtungen (zum Beispiel Rasieren, Waschen, Essen mit Messer und Gabel, Schreiben etc.) wieder selbständig. Dieser Effekt hielt über mehrere Tage nach Therapieende an und klang dann im Verlaufe der folgenden Wochen allmählich ab.

Drei Weitere ALS-Patienten, die seit 5 bzw. 7 bzw. 12 Monaten erkrankt waren, erhielten an vier Tagen dreimal täglich je 24 mg Orotyl-L-histidyl-L-prolinamid, gelöst in 5 %-iger wässriger Glucoselösung innerhalb von jeweils 30 Minuten intravenös infundiert. Es waren deutliche Besserungen in der Benutzungsmöglichkeit von Knöpfen und Reißverschlüssen sowie beim Gehen ohne Hilfe zu beobachten. Die clonischen Reflexe normalisierten sich, und die schnelle Ermüdbarkeit der Patienten wurde herabgesetzt. Auch bei diesen Patienten wurden nur unbedeutende und zu vernachlässigende Nebenwirkungen beobachtet.

Bei einer Patientin mit ausgeprägtem ALS-bedingten Schwund der Handmuskulatur bewirkte bereits die Verabreichung von insgesamt 18 mg Orotyl-L-histidyl-L-prolinamid an nur einem Tag (durch Infusion von je 6 mg in Abständen von 4 Stunden) einen deutlichen günstigen Effekt auf die Handmuskulatur, der auch nach Absetzen der Medikation noch einige Zeit anhielt.

Ähnliche Resultate wurden auch bei Verabreichung anderer Arzneimittel, die das Orotyl-L-histidyl-L-prolinamid enthielten sowie durch Applikation anderer Verbindungen der allgemeinen Formel I in entsprechenden pharmazeutischen Zubereitungsformen an ALS-Patienten beobachtet.

## Ansprüche

1. Verwendung von Dipeptidderivaten der allgemeinen Formel I

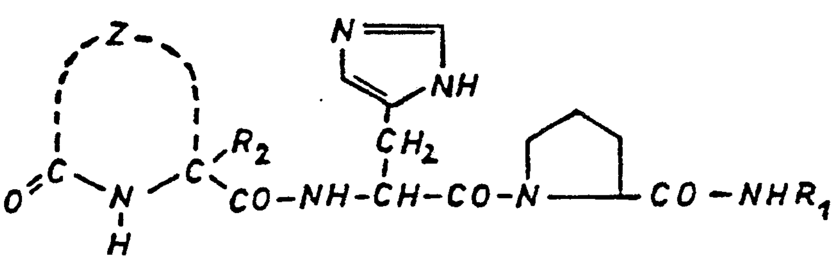

(I)

in der $R_1$ ein Wasserstoffatom, einen Alkylrest mit bis zu 6 Kohlenstoffatomen, einen Cyclohexylrest oder einen Benzylrest bedeutet, und in der Z für eine der (mit der durch den Stern markierten Bindung an die CO-Gruppe im Ring angreifenden) Gruppen

(a) 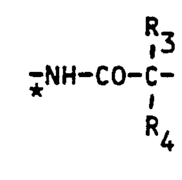 oder (b) 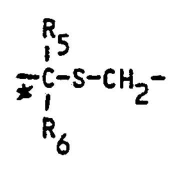

steht, $R_2$ zusammen mit $R_3$ für eine zusätzliche Bindung oder, wenn Z die Gruppe (b) darstellt, für ein Wasserstoffatom steht und in der $R_4$ und $R_5$ für Wasserstoffatome oder für Alkylreste mit 1 bis 3

Kohlenstoffatomen stehen und $R_5$ auch Phenyl bedeuten kann und $R_6$ ein Wasserstoff atom oder Methyl bedeutet
oder deren pharmazeutisch anwendbaren Salzen mit Säuren
für die Herstellung von Arzneimitteln zur Behandlung von an amyotropher Lateralsklerose Erkrankten.

**2.** Verwendung von Dipeptidderivaten der allgemeinen Formel

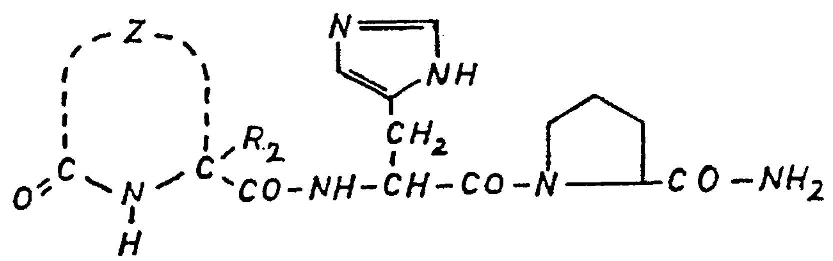

in der $R_2$ und Z die gleiche Bedeutung wie in Anspruch 1 haben
oder deren pharmazeutisch anwendbaren Salzen mit Säuren
für die Herstellung von Arzneimitteln zur Behandlung von an amyotropher Lateralsklerose Erkrankten gemäß Anspruch 1.

**3.** Verwendung von Dipeptidderivaten der allgemeinen Formel Ia

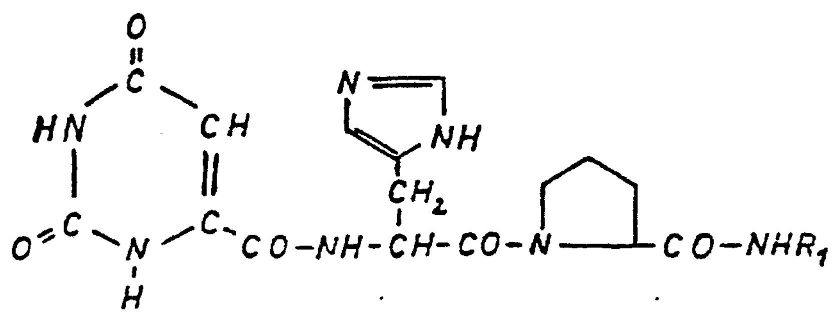

(Ia)

worin $R_1$ die gleiche Bedeutung wie in Anspruch 1 hat, oder deren pharmazeutisch anwendbaren Salzen mit Säuren
für die Herstellung von Arzneimitteln zur Behandlung von an amyotropher Lateralsklerose Erkrankten gemäß Ansprüchen 1 und 2.

**4.** Verwendung von Dipeptidderivaten der allgemeinen Formel Ib

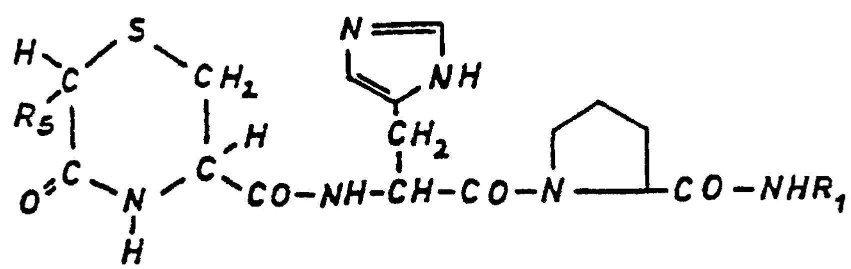

(Ib)

worin $R_1$ und $R_5$ die gleiche Bedeutung wie in Anspruch 1 haben
oder deren pharmazeutisch anwendbaren Salzen mit Säuren
für die Herstellung von Arzneimitteln zur Behandlung von an amyotropher Lateralsklerose Erkrankten gemäß Ansprüchen 1 und 2.

**5.** Verwendung von Orotoyl-L-histidyl-L-prolinamid oder eines von dessen pharmazeutisch anwendbaren

Salzen mit Säuren für die Herstellung von Arzneimitteln zur Behandlung von an amyotropher Lateralsklerose Erkrankten gemäß Ansprüchen 1 - 3.

6. Verwendung von 5-Oxo-6-methyl-thiomorpholin-3-(L)-carbonyl-L-histidyl-L-prolinamid oder eines von dessen pharmazeutisch anwendbaren Salzen mit Säuren für die Herstellung von Arzneimitteln zur Behandlung von an amyotroher Lateralsklerose Erkrankten gemäß Ansprüchen 1, 2 und 4.

7. Verwendung von Dipeptidderivaten der allgemeinen Formeln aus Ansprüchen 1 bis 4 oder deren pharmazeutisch anwendbaren Salzen mit Säuren für die Herstellung von Arzneimitteln zur Behandlung in Form von Infusionen oder Injektionen von an amyotropher Lateralsklerose Erkrankten.

8. Verwendung von Dipeptidderivaten der allgemeinen Formeln aus Ansprüchen 1 bis 4 oder deren pharmazeutisch anwendbaren Salzen mit Säuren für die Herstellung von Arzneimitteln in oral oder rektal anzuwendender Form zur Behandlung von an amyotropher Lateralsklerose Erkrankten.

9. Verwendung von Dipeptidderivaten der allgemeinen Formeln aus Ansprüchen 1 bis 4 oder deren pharmazeutisch anwendbaren Salzen mit Säuren für die Herstellung von Arzneimitteln zur percutanen Wirkstoffzuführung für die Behandlung von an amyotropher Lateralsklerose Erkrankten.

## Claims

1. Use of dipeptide derivatives of general formula I

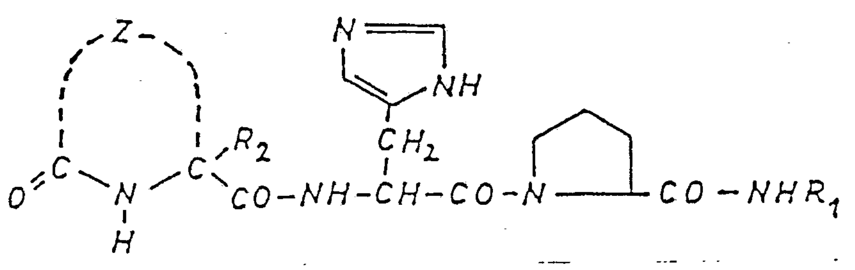

(I)

in which $R_1$ signifies a hydrogen atom, an alkyl group with up to 6 carbon atoms, a cyclohexyl group or a benzyl group, and in which Z represents one of the groups

(a) 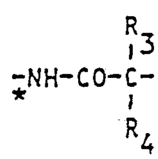 or (b) 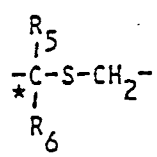

(attached to the CO group in the ring by the bond marked with an asterisk), $R_2$ either together with $R_3$ represents an additional bond or, if Z represents the group (b), represents a hydrogen atom, and in which $R_4$ and $R_5$ represent hydrogen atoms or alkyl groups with 1 to 3 carbon atoms and $R_5$ can also signify phenyl and $R_6$ signifies a hydrogen atom or methyl
or their pharmaceutically applicable salts with acids
for the production of drugs for the treatment of sufferers from amyotrophic lateral sclerosis.

2. Use of dipeptide derivatives of general formula

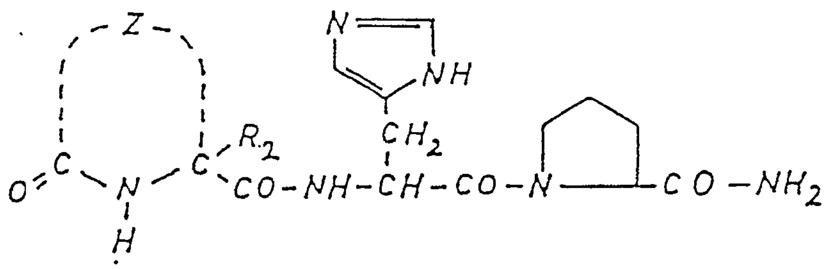

in which $R_2$ and Z have the same significance as in Claim 1 or their pharmaceutically applicable salts with acids for the production of drugs for the treatment of sufferers from amyotrophic lateral sclerosis according to Claim 1.

3. Use of dipeptide derivatives of general formula Ia

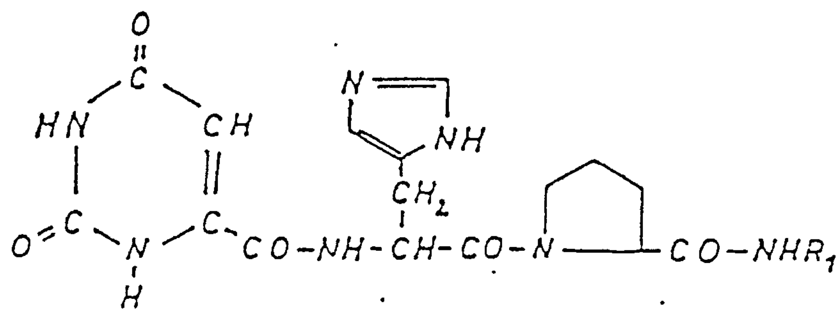

(Ia)

in which $R_1$ has the same significance as in Claim 1 or their pharmaceutically applicable salts with acids for the production of drugs for the treatment of sufferers from amyotrophic lateral sclerosis according to Claims 1 and 2.

4. Use of dipeptide derivatives of general formula Ib

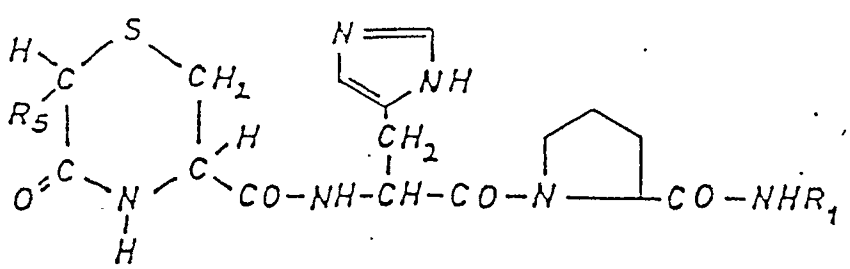

(Ib)

in which $R_1$ and $R_5$ have the same significance as in Claim 1 or their pharmaceutically applicable salts with acids for the production of drugs for the treatment of sufferers from amyotrophic lateral sclerosis according to Claims 1 and 2.

5. Use of orotyl L-histidyl-L-prolinamide or of one of its pharmaceutically applicable salts with acids for the production of drugs for the treatment of sufferers from amyotrophic lateral sclerosis according to Claims 1-3.

6. Use of 5-oxo-6-methyl-thiomorpholine-3-(L)-carbonyl-L-histidyl-L-prolinamide or of one of its pharmaceutically applicable salts with acids for the production of drugs for the treatment of sufferers from amyotrophic lateral sclerosis according to Claims 1, 2 and 4.

7. Use of dipeptide derivatives of the general formulae of Claims 1 to 4 or their pharmaceutically applicable salts with acids for the production of drugs for the treatment in the form of infusions or injections of sufferers from amyotrophic lateral sclerosis.

8. Use of dipeptide derivatives of the general formulae of Claims 1 to 4 or their pharmaceutically applicable salts with acids for the production of drugs in forms for oral or rectal application for the treatment of sufferers from amyotrophic lateral sclerosis.

9. Use of dipeptide derivatives of the general formulae of Claims 1 to 4 or their pharmaceutically applicable salts with acids for the production of drugs for percutaneous feeding of active substance for the treatment of sufferers from amyotrophic lateral sclerosis.

## Revendications

1. Utilisation de dérivés dipeptidiques de formule générale I

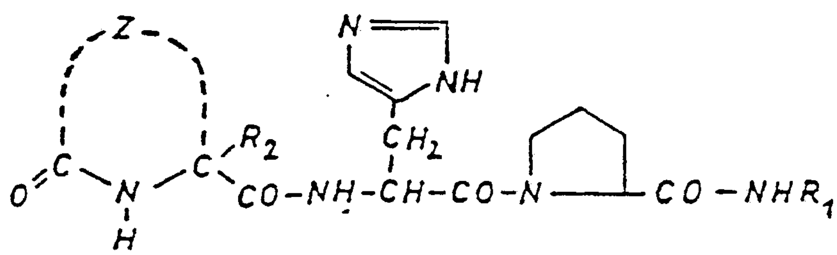

(I)

dans laquelle $R_1$ est un atome d'hydrogène, un reste alkyle ayant jusqu'à 6 atomes de carbone, un reste cyclohexyle ou un reste benzyle et dans laquelle Z représente l'un des groupes (s'attachant au groupe CO du noyau par la liaison marquée de l'astérisque)

(a) 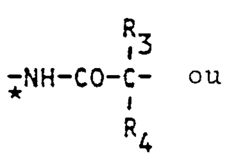 ou (b) 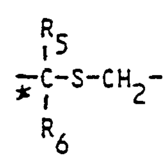

$R_2$ forme conjointement avec $R_3$ une liaison supplémentaire ou bien, lorsque Z représente le groupe (b), il représente un atome d'hydrogène, et $R_4$ et $R_5$ représentent des atomes d'hydrogène ou des restes alkyle ayant 1 à 3 atomes de carbone et $R_5$ peut aussi représenter un groupe phényle et $R_6$ désigne un atome d'hydrogène ou un groupe méthyle,
ou de leurs sels d'acides acceptables du point de vue pharmaceutique
pour la préparation de médicaments destinés au traitement de malades atteints de sclérose latérale amyotrophique.

2. Utilisation de dérivés dipeptidiques de formule générale

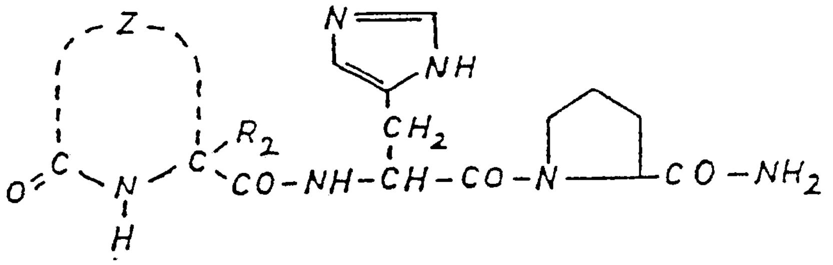

dans laquelle $R_2$ et Z ont la même définition que dans la revendication 1 ou de leurs sels d'acides acceptables du point de vue pharmaceutique pour la préparation de médicaments destinés au traitement de malades atteints de sclérose latérale amyotrophique, suivant la revendication 1.

3. Utilisation de dérivés dipeptidiques de formule générale Ia

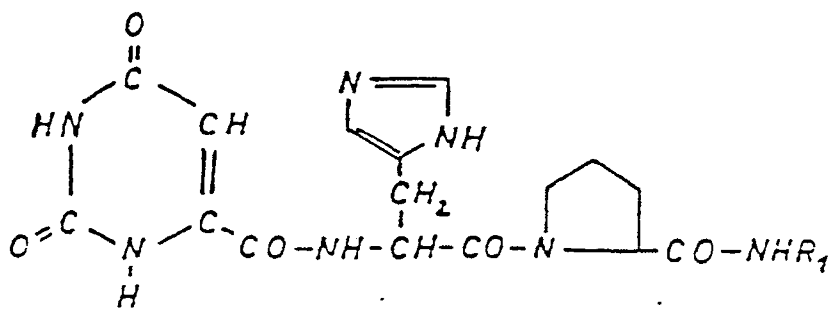

(Ia)

dans laquelle $R_1$ a la même définition que dans la revendication 1, ou de leurs sels d'acides acceptables du point de vue pharmaceutique, pour la préparation de médicaments destinés au traitement de malades atteints de sclérose latérale amyotrophique, suivant les revendications 1 et 2.

4. Utilisation de dérivés dipeptidiques de formule générale Ib

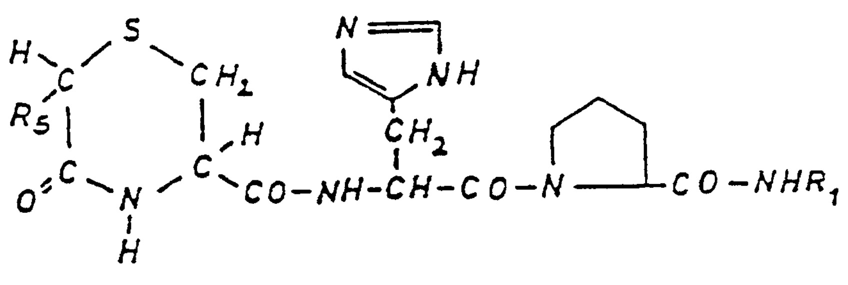

(Ib)

dans laquelle $R_1$ et $R_5$ ont la même définition que dans la revendication 1 ou de leurs sels d'acides acceptables du point de vue pharmaceutique pour la préparation de médicaments destinés au traitement de malades atteints de sclérose latérale amyotrophique, suivant les revendications 1 et 2.

5. Utilisation d'orotyl-L-histidyl-L-prolinamide ou de l'un de ses sels d'acides acceptables du point de vue pharmaceutique pour la préparation de médicaments destinés au traitement de malades atteints de sclérose latérale amyotrophique, suivant les revendications 1 à 3.

6. Utilisation du 5-oxo-6-méthyl-thiomorpholine-3(L)-carbonyl-L-histidyl-L-prolinamide ou de l'un de ses

sels d'acides acceptables du point de vue pharmaceutique pour la préparation de médicaments destinés au traitement de malades atteints de sclérose latérale amyotrophique, suivant les revendications 1, 2 et 4.

**7.** Utilisation de dérivés dipeptidiques de formules générales suivant les revendications 1 à 4 ou de leurs sels d'acides acceptables du point de vue pharmaceutique pour la préparation de médicaments sous forme d'infusions ou d'injections pour le traitement de malades atteints de sclérose latérale amyotrophique.

**8.** Utilisation de dérivés dipeptidiques de formules générales suivant les revendications 1 à 4 ou de leurs sels d'acides acceptables du point de vue pharmaceutique pour la préparation de médicaments sous forme orale ou rectale destinés au traitement de malades atteints de sclérose latérale amyotrophique.

**9.** Utilisation de dérivés dipeptidiques de formules générales suivant les revendications 1 à 4 ou de leurs sels d'acides acceptables du point de vue pharmaceutique pour la préparation de médicaments introduisant la substance active par voie percutanée, pour le traitement de malades atteints de sclérose latérale amyotrophique.